(19) Europäisches Patentamt
European Patent Office
Office européen des brevets

(11) Veröffentlichungsnummer: **0 344 113 A2**

# (12) EUROPÄISCHE PATENTANMELDUNG

(21) Anmeldenummer: **89810368.4**

(22) Anmeldetag: **18.05.89**

(51) Int. Cl.[4]: **C 07 D 309/38**
C 07 C 69/734, C 07 C 50/24,
C 07 C 50/30

(30) Priorität: **27.05.88 CH 2008/88**
**19.07.88 CH 2753/88**

(43) Veröffentlichungstag der Anmeldung:
**29.11.89 Patentblatt 89/48**

(84) Benannte Vertragsstaaten:
**CH DE FR GB IT LI NL**

(71) Anmelder: **CIBA-GEIGY AG**
**Klybeckstrasse 141**
**CH-4002 Basel (CH)**

(72) Erfinder: **Kvita, Vratislav, Dr.**
**Vogesenstrasse 71**
**CH-4153 Reinach (CH)**

**Baumann, Marcus, Dr.**
**Arlesheimerstrasse 18**
**CH-4053 Basel (CH)**

**Fischer, Walter, Dr.**
**Vogesenstrasse 77**
**CH-4153 Reinach (CH)**

**Mayer, Carl W., Dr.**
**Steingrubenweg 224**
**CH-4125 Riehen (CH)**

**Allmendinger, Thomas, Dr.**
**Schloss-Strasse 32/7**
**D-7853 Steinen (DE)**

(54) **Substituierte alpha-Pyrone und Naphthochinone.**

(57) α-Pyrone der Formel I

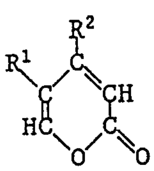

(I),

worin
$R^1$ für $-CF_3$ oder $-COOR^3$ steht und $R^3$ den um eine Hydroxylgruppe verminderten Rest eines $C_1$-$C_{18}$-Alkohols bedeutet, und $R^2$ -F, -Br, -Cl, -CN, $-CF_3$, $C_1$-$C_{18}$-Alkyl, $C_2$-$C_{18}$-Alkenyl oder -Alkinyl, $C_1$-$C_{18}$-Alkoxy, $C_1$-$C_{18}$-Alkylthio, $C_1$-$C_{18}$-Alkylsulfonyl, $C_6$-$C_{16}$-Aryl, $C_7$-$C_{24}$-Alkaryl, $C_7$-$C_{12}$-Aralkyl, $C_8$-$C_{24}$-Alkaralkyl, $C_6$-$C_{16}$-Aryloxy oder -thio oder -sulfonyl, $C_7$-$C_{24}$-Alkaryloxy oder -thio oder -sulfonyl, $C_7$-$C_{12}$-Aralkyloxy oder -thio oder -sulfonyl oder $C_8$-$C_{24}$-Alkaralkyloxy oder -thio oder -sulfonyl, Sekundäramino mit 2 bis 24 C-Atomen, oder Trialkyl- oder Trialkoxysilyl mit 3 bis 18 C-Atomen darstellt. Sie eignen sich zur Herstellung von Naphto-und Anthra-1,4-chinonen, aus denen Tetrathio- oder Tetraselenotetracenen erhältlich sind, die elektrochrome Eigenschaften aufweisen.

EP 0 344 113 A2

**Beschreibung**

## Substituierte α-Pyrone und Naphthochinone

Die Erfindung betrifft 4-substituierte α-Pyron-5-carbonsäureester oder 5-Trifluormethyl-α-pyrone und 7-substituierte Naphtho-1,4-chinon-6-carbonsäureester bzw. 6-Trifluormethyl-naphtho-1,4-chinone sowie substituierte Butadiendicarbonsäureester.

α-Pyron-5-carbonsäureester sind bekannt, siehe zum Beispiel H. Gault et al., C.r. Akad. Sci, Paris Ser. C, 266, S. 131-134 (1968). In der US-PS 4,617,151 sind α-Pyron-4,5-dicarbonsäurediester beschrieben.

Andere in 3,6-Stellung unsubstituierte und in 4-Stellung substituierte α-Pyron-5-carbonsäureester sind nicht bekannt. Es wurde nun gefunden, dass solche α-Pyrone in einfacher Weise durch Umsetzung von Allendicarbonsäureestern mit metallorganischen Verbindungen zu in 2-Stellung substituierten Propendicarbonsäurediestern, deren Umsetzung mit Ameisensäureestern zu in 3-Stellung substituierten 1-Alkoxymethylen-butadien-2,4-dicarbonsäureestern und deren Cyclisierung zu α-Pyronen erhältlich sind.

Ein Gegenstand der Erfindung sind Verbindungen der Formel I

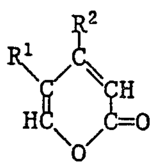

(I),

worin

$R^1$ für $-CF_3$ oder $-COOR^3$ steht und $R^3$ den um eine Hydroxylgruppe verminderten Rest eines $C_1$-$C_{18}$-Alkohols bedeutet, und $R^2$ -F, -Br, -Cl, -CN, $-CF_3$, $C_1$-$C_{18}$-Alkyl, $C_2$-$C_{18}$-Alkenyl oder -Alkinyl, $C_1$-$C_{18}$-Alkoxy, $C_1$-$C_{18}$-Alkylthio, $C_1$-$C_{18}$-Alkylsulfonyl, $C_6$-$C_{16}$-Aryl, $C_7$-$C_{24}$-Alkaryl, $C_7$-$C_{12}$-Aralkyl, $C_8$-$C_{24}$-Alkaralkyl, $C_6$-$C_{16}$-Aryloxy oder -thio oder -sulfonyl, $C_7$-$C_{24}$-Alkaryloxy oder -thio oder -sulfonyl, $C_7$-$C_{12}$-Aralkyloxy oder -thio oder -sulfonyl oder $C_8$-$C_{24}$-Alkaralkyloxy oder -thio oder -sulfonyl, Sekundäramino mit 2 bis 24 C-Atomen, oder Trialkyl- oder Trialkoxysilyl mit 3 bis 18 C-Atomen darstellt.

In einer bevorzugten Untergruppe steht $R^2$ für -F, -Br, -Cl, $-CF_3$, $C_1$-$C_{18}$-Alkyl, $C_2$-$C_{18}$-Alkenyl, $C_1$-$C_{18}$-Alkoxy, $C_1$-$C_{18}$-Alkylthio, $C_1$-$C_{18}$-Alkylsulfonyl, $C_6$-$C_{10}$-Aryl, $C_7$-$C_{24}$-Alkaryl, $C_7$-$C_{12}$-Aralkyl, $C_6$-$C_{10}$-Aryloxy oder -thio oder -sulfonyl, $C_7$-$C_{12}$-Aralkyloxy oder -thio oder -sulfonyl.

$R^3$ als Rest eines Alkoholes enthält bevorzugt 1-12, besonders 1 bis 6 C-Atome. $R^3$ kann z.B. lineares oder verzweigtes Alkyl, gegebenenfalls mit $C_1$-$C_6$-Alkyl substituiertes $C_5$- oder $C_6$-Cycloalkyl, oder gegebenenfalls mit $C_1$-$C_6$-Alkyl substituiertes $C_6$-$C_{12}$-Aryl oder $C_6$-$C_{12}$-Aryl-$C_xH_{2x}$ mit x gleich 1 bis 4 sein. Aryl ist bevorzugt Phenyl und x ist bevorzugt 1. Bevorzugt stellt $R^3$ $C_1$-$C_{18}$-, besonders $C_1$-$C_{12}$- und besonders $C_1$-$C_6$-Alkyl dar. Einige Beispiele sind Methyl, Ethyl, die Isomeren von Propyl, Butyl, Pentyl, Hexyl, Heptyl, Octyl, Nonyl, Decyl, Undecyl, Dodecyl, Tetradecyl, Hexadecyl, Octadecyl, Cyclopentyl, Cyclohexyl, Methylcyclohexyl, Phenyl, Benzyl, Methylphenyl, Dimethylphenyl, Ethylphenyl, Methylethylphenyl, t-Butylphenyl und Methylbenzyl. Besonders bevorzugt stellt $R^3$ Methyl oder Ethyl dar.

$R^2$ als Alkyl, Alkoxy, Alkylthio und Alkylsulfonyl kann linear oder verzweigt sein und enthält bevorzugt 1 bis 12, besonders 1 bis 6 und insbesondere 1 bis 4 C-Atome. Beispiele sind Methyl, Ethyl, die Isomeren von Propyl, Butyl, Pentyl, Hexyl, Heptyl, Octyl, Nonyl, Decyl, Undecyl, Dodecyl, Tridecyl, Tetradecyl, Pentadecyl, Heptadecyl, Octadecyl und entsprechende Alkoxy-, Alkylthio- und Alkylsulfonylreste.

$R^2$ als Alkenyl und Alkinyl kann linear oder verzweigt sein und enthält bevorzugt 2 bis 12, besonders 2 bis 6 C-Atome. Einige Beispiele sind Vinyl, Ethinyl, Allyl, Propargyl, Prop-1-en-1-yl, But-1-en- oder -2-en-4-yl, Pent-2-en-5-yl, Hex-3-en-6-yl, But-2-in-4-yl, Pent-2-in-4- oder -5-yl und Hex-3-in-5- oder -6-yl.

Stellt $R^2$ Aryl oder einen Aryl enthaltenden Rest dar, so ist das Aryl z.B. Naphthyl oder besonders Phenyl. $R^2$ in der Bedeutung von Alkaryl und entsprechende Oxy-, Thio- und Sulfonylreste enthalten bevorzugt 7 bis 18 C-Atome. $R^2$ in der Bedeutung von Aralkyl und entsprechende Oxy-, Thio-und Sulfonylreste enthalten bevorzugt 7 oder 8 C-Atome. $R^2$ in der Bedeutung von Alkaralkyl und entsprechende Oxy-, Thio- und Sulfonylreste enthalten bevorzugt 8 bis 20 C-Atome. In einer bevorzugten Untergruppe stellt $R^2$ in der Bedeutung von Aryl oder einem Aryl enthaltenden Rest Phenyl, Phenyloxy, Phenylthio oder Phenylsulfonyl, $C_1$-$C_{12}$-Alkylphenyl, -phenyloxy, -phenylthio oder -phenylsulfonyl, $C_1$-$C_{12}$-Alkylphenyl-$C_nH_{2n}$-, $-C_nH_{2n}O$-, $C_nH_{2n}S$- oder $-C_nH_{2n}SO_2$-, oder Phenyl-$C_nH_{2n}$-, $-C_nH_{2n}O$-, $-C_nH_{2n}S$- oder $-C_nH_{2n}SO_2$- dar, worin n eine Zahl von 1 bis 4, besonders 1 oder 2 und insbesondere 1 ist. Einige Beispiele sind Phenyl, Phenyloxy, Phenylthio, Benzyl, Benzyloxy, Benzylthio, Methylphenyl, Methylphenyloxy, Methylphenylthio, Methylbenzyl, Methylbenzyloxy, Methylbenzylthio.

$R^2$ enthält in der Bedeutung von Sekundäramino bevorzugt 2 bis 18, besonders 2 bis 12 und insbesondere 2 bis 8 C-Atome. Das Sekundäramino kann der Formel $-NR^5R^6$ entsprechen, worin $R^5$ und $R^6$ unabhängig voneinander lineares oder verzweigtes $C_1$-$C_{18}$-, besonders $C_1$-$C_{12}$- und insbesondere $C_1$-$C_6$-Alkyl, Cyclopentyl, Cyclohexyl, Phenyl, Benzyl, $C_1$-$C_6$-Alkylphenyl, $C_1$-$C_6$-Alkylbenzyl oder $R^5$ und $R^6$ zusammen Tetra- oder Pentamethylen oder 3-Oxa-1,5-pentylen bedeuten. Einige Beispiele sind Dimethyl-, Diethyl-, Ethylmethyl-, Dibutyl-, Dioctyl-, Methyldodecyl-, Didodecyl-, Methyloctadecyl-, Phenylmethyl-, Benzylmethylamino, Pyrrolino, Piperidino und Morpholino.

$R^2$ enthält als Trialkylsilyl oder Trialkoxysilyl bevorzugt 3 bis 12, besonders 3 bis 8 C-Atome. Alkyl- und Alkoxygruppen sind zuvor erwähnt worden. Einige Beispiele sind Trimethyl-, Triethyl-, Dimethylethyl-, Tributyl-, Dimethylbutyl-, Dimethyl-(1,1,2,2-tetramethylethyl)-, Dimethyloctyl-, Trimethoxy- und Triethoxysilyl.

Eine bevorzugte Gruppe von erfindungsgemässen Verbindungen sind solche, worin $R^2$ für -F, -CN, $-CF_3$, -Br, -Cl, $C_1$-$C_{18}$-Alkyl oder $C_2$-$C_{12}$-Alkenyl, $C_1$-$C_{18}$-Alkoxy, $C_1$-$C_{18}$-Alkylthio, $C_1$-$C_{18}$-Alkylsulfonyl, Phenyl, $C_1$-$C_{12}$-Alkylphenyl, Phenyl-$C_nH_{2n}$-, $C_1$-$C_{12}$-Alkylphenyl-$C_n$ $H_{2n}$-, Phenyloxy, Phenyl thio oder Phenylsulfonyl, $C_1$-$C_{12}$-Alkylphenoxy, -phenylthio oder -phenylsulfonyl, Phenyl-$C_nH_{2n}$-O-, Phenyl-$C_nH_{2n}$-S- oder Phenyl-$C_nH_{2n}$-$SO_2$-, $C_1$-$C_{12}$-Alkylphenyl-$C_nH_{2n}$-O-, $C_1$-$C_{12}$-Alkylphenyl-$C_nH_{2n}$-S-oder $C_1$-$C_{12}$-Alkylphenyl-$C_nH_{2n}$-$SO_2$-, wobei n eine Zahl von 1 bis 4 ist, Sekundäramino mit 2 bis 18 C-Atomen, oder Trialkyl- oder Trialkoxysilyl mit 3 bis 12 C-Atomen steht.

In einer besonders bevorzugten Ausführungsform steht $R^2$ für -F, -Cl, -Br, $-CF_3$, $C_1$-$C_{12}$-Alkyl, $C_1$-$C_{12}$-Alkoxy, $C_1$-$C_{12}$-Alkylthio, Benzylthio oder $C_1$-$C_{12}$-Alkylbenzylthio, oder Sekundäramino mit 2 bis 12 C-Atomen.

Ein weiterer Gegenstand der Erfindung ist ein Verfahren zur Herstellung von Verbindungen der Formel I, das dadurch gekennzeichnet ist, dass man
eine Verbindung der Formel II

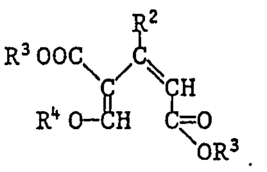

(II),

worin $R^2$ und $R^3$ die zuvor angegebenen Bedeutungen haben, und $R^4$ für $C_1$-$C_4$-Alkyl steht, in Gegenwart einer starken wasserfreien Säure zu einer Verbindung der Formel I cyclisiert.

In Formel II steht $R^4$ besonders für Methyl oder Ethyl.

Die Verbindungen der Formel II sind ein weiterer Gegenstand der Erfindung. Sie können in analoger Weise nach dem in der US-PS 4,617,151 beschriebenen Verfahren hergestellt werden durch Umsetzung von Propen-1,3-dicarbonsäurediestern der Formeln III

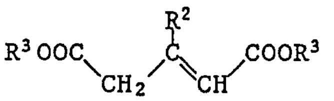

(III)

mit Ameisensäureestern $HCOOR^4$ in Gegenwart von $TiCl_4$, einem Alkalimetallhydrid oder -alkoholat. Geeignete Alkalimetalle sind z.B. Li, Na und K und Beispiele für Alkohole $C_1$-$C_4$-Alkanole, z.B. Methanol und Ethanol.

Die Verbindungen der Formel III können auf unterschiedliche Weise hergestellt werden. Ausgangsverbindung ist z.B. der bekannte Ketodicarbonsäurediester der Formel IV

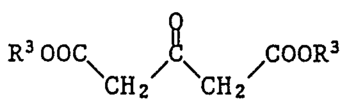

(IV)

Durch Enolisierung mit Alkylierungsmitteln, z.B. Dialkylsulfaten, Orthoameisensäureestern in Gegenwart von $FeCl_3$, oder Alkylhalogeniden erhält man Verbindungen der Formel III, worin $R^2$ einen Alkoxyrest bedeutet.

Die Umsetzung der Verbindung der Formel IV mit z.B. $PCl_5$ oder Diethylaminoschwefeltrifluorid (DAST) ergibt Verbindungen der Formel III, worin $R^2$ Cl oder F bedeutet. Diese können mit Alkalimetallsalzen von organischen Mercaptoverbindungen bzw. Sulfonylverbindungen (Alkalimetall ist z.B. Li, Na, K) zu Verbindungen der Formel III umgesetzt werden, worin $R^2$ einen der zuvor definierten Thioreste bzw. Sulfonylresten bedeutet.

Verbindungen der Formel III mit $R^2$ = Cl können in üblicher Weise zu Allendicarbonsäureestern der Formel V

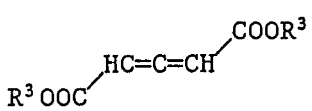

(V)

dehydrochloriert werden. Deren Umsetzung mit $NH_4HF_2$ oder (n-Butyl)$_4NH_2F_3$ führt auch zu Verbindungen der Formel III, worin $R^2$ F ist. Mit metallorganischen Reagenzien $R^2Z$ ($R^2$ ist nicht Halogen oder $-CF_3$), worin Z vorzugsweise ein Alkalimetall, insbesondere Li, Na oder K ist, und anschliessende Hydrolyse erhält man Verbindungen der Formel III. Wenn $R^2$ ein Kohlenwasserstoffrest ist, wird bei der Umsetzung mit $R^2Z$ vorzugsweise ein anorganisches Kupfersalz mitverwendet, z.B. Kupfer(I)halogenide oder besonders CuCN. Sekundäramino-substituierte Verbindungen der Formel III erhält man auch durch direkte Umsetzung mit

sekundären Aminen.

Die Umsetzung mit $R^2Z$ wird vorteilhaft in einem polaren aprotischen Lösungsmittel durchgeführt. Beispiele sind Ether, insbesondere (Diethylether, Dibutylether, Tetrahydrofuran, Dioxan, Ethylenglykoldiethylether). Die Reaktionstemperatur beträgt zweckmässig -20 bis -100°C.

Verbindungen der Formel I, worin $R^1$ und/oder $R^2$ $-CF_3$ bedeuten, erhält man durch Fluorierung, z.B. mit $SF_4/HF$, der entsprechenden Carbonsäurealkylester, besonders der -methyl- oder -ethylester.

Verbindungen der Formel I, worin $R^2$ einen Thio- oder Sulfonylrest bedeuten, können auch durch entsprechende Substitution von α-Pyronen der Formel I mit $R^2$ gleich Cl oder Br hergestellt werden.

Die Cyclisierung von Verbindungen der Formel II kann ebenfalls nach dem in der US-PS 4,617,151 beschriebenen Verfahren erfolgen. Vorteilhaft wird die Cyclisierung mit wasserfreier Ameisensäure oder Polyphosphorsäure ohne Lösungsmittel durchgeführt. Die Temperatur für diese Reaktion beträgt zweckmässig 50 bis 250°C.

Die Verbindungen der Formel I können mit Benzo-1,4-chinon zu Naphthochinonen der Formel VI

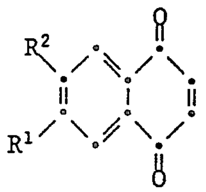

(VI),

umgesetzt werden, worin $R^1$ und $R^2$ die in Formel I angegebenen Bedeutungen haben. Diese Naphtochinone sind ein weiterer Gegenstand der Erfindung.

Vorzugsweise bedeutet $R^2$ in Formel VI $-CF_3$, F, -Cl, -Br, $C_1$-$C_{18}$-Alkyl, -Alkoxy oder -Alkylthio oder -Alkylsulfonyl, Phenyloxy oder -thio oder -sulfonyl, $C_1$-$C_{12}$-Alkylphenyloxy oder -thio oder -sulfonyl, Benzyloxy oder -thio oder -sulfonyl, oder $C_1$-$C_{12}$-Alkylbenzyloxy oder -thio oder -sulfonyl. In einer besonderen Ausführungsform stehen $R^1$ für $-CF_3$ oder $-COOR^3$ und $R^2$ für $-CF_3$, -F, -Cl, $C_1$-$C_{12}$-Alkyl, -Alkoxy oder -Alkylthio oder -Alkylsulfonyl, oder Benzylthio und $R^3$ für $C_1$-$C_4$-Alkyl.

Die Verbindungen der Formel I und VI sind wertvolle Zwischenprodukte zur Herstellung von substituierten Anthracen-5,12-dionen, aus denen substituierte Tetrathio- bzw. Tetraselenotetracenen erhältlich sind (vgl. US-PS 4 617 151). Aus solchen chalkogenierten Tetracenen können mit Elektronenacceptoren elektrisch leitende Charge-Transferkomplexe (CT-Komplexe) hergestellt werden. Mit deren funktionellen Gruppen kann man sie an Polymere binden, z.B. als Seitengruppen in Polymere einbauen (vgl. US-PS 4,617,151). Die CT-Komplexe eignen sich auch zur Herstellung z.B. von antistatischen Beschichtungen photographischer Filmelemente, Magnetbänder, elektrophotographischer Filmelemente und elektronischer Komponenten (siehe US-PS 3,634,336). Die chalkogenierte Tetracene weisen ferner elektrochrome Eigenschaften auf; sie können für elektrochrome Displays verwendet werden. Sie eignen sich auch als laser-optische Datenspeicher [Nach. Chem. Techn. Lab. 35, S. 255 ff (1987)] und als Anodenmaterial in organischen Solid-State-Batterien (EP-A-0 090 598). CT-Komplexe von unsubstituierten oder substituierten Tetrathio- oder Tetraselenotetracenen können zur Erzielung antistatischer Eigenschaften auch in thermoplastische, duroplastische oder elastomere Polymere eingearbeitet werden. Hierzu werden zweckmässig z.B. diese Tetrathio-bzw. Tetraselenotetracene zusammen mit einem löslichen Polymer oder einer Vorstufe davon und einem Elektronenacceptor, z.B. einem Halogen bildenden Mittel (organische halogenierte Verbindungen wie z.B. Bromoform, Trichlorbrommethan, Tetrabrommethan, Hexachlorpropan, Perchlorbutadien, 1,3-oder 1,4-Dichlor-2-buten, 1,4-Bis(trichloromethyl)-benzol, Iodacetonitril, Iodoform, Tetrachlorethylen, Perchlorcyclobutadien, N-Chlor-, -brom oder -iodsuccinimid) gegebenenfalls zusammen mit einem weiteren inerten Lösungsmittel gelöst und das überschüssige Halogen bildende Mittel und das Lösungsmittel bei höherer Temperatur verdampft. Die gebildete Zusammensetzung enthält im Polymer ein Netzwerk nadelförmiger Kristalle des CT-Komplexes, wenn das chalkogenierte Tetracen unsubstituiert ist oder kleine Substituenten (z.B. F, $CH_3$, $CF_3$) enthält. Solche Zusammensetzungen weisen eine hohe elektrische Leitfähigkeit auf. Diese kann noch verbessert werden, wenn man ein aus den Verbindungen der Formel I hergestelltes substituiertes Tetrathio-oder Tetraselenotetracen mitverwendet, das kein solches Netzwerk bildet und das in der Polymermatrix in feiner Verteilung vorliegt, da solche substituierten Tetrathio-oder Tetraselenotetracene keine oder nur eine geringe Kristallisationstendenz im Polymer besitzen.

Die Verbindungen der Formel I sind wertvolle Dienkomponenten für Diels-Alder-Reaktionen, wobei unterschiedliche Dienophile verwendet werden können. Bei Verwendung von gegebenenfalls substituierten 1,4-Chinonen, z.B. Benzo-1,4-chinon, Naphthalin-1,4-chinon oder Anthracen-1,4-chinon, erhält man um einen Ring erweiterte Chinone (siehe z.B. EP-A-0 195 743). Substituierte Naphthacen-5,12-dione sind auch direkt erhältlich durch die Umsetzung von gegebenenfalls substituierten Naphthalin-1,4-chinonen mit in 1- und 2-Stellung mit Brom und/oder Iod substituierten Benzocyclobutenen. Anthrachinone und Naphthacendione können z.B. als Photosensibilisatoren (vgl. US-PS 3,941,759) oder Photoinitiatoren zur Polymerisation ethylenisch ungesättigter Verbindungen verwendet werden, besonders mindestens in 2-Stellung mit einem Thiorest substituierte Naphthacen-5,12-dione. Naphthacenchinone können auch in elektrochromen Displayelementen verwendet werden (JP-OS 61-43680).

Die nachfolgenden Beispiele erläutern die Erfindung.

Beispiel 1: 4-Butyl-2H-2-oxo-pyran-5-carbonsäureethylester

a) 2-Butyl-prop-1-en-1,3-dicarbonsäure-diethylester

10g CuCN werden in 200 ml Toluol aufgeschlämmt und das Toluol bei 35°C am Rotationsverdampfer abdestilliert. Der Vorgang wird nochmals wiederholt. Das Vakuum wird durch Argon ersetzt, 200 ml Tetrahydrofuran (THF) zugefügt und die Suspension auf -70°C abgekühlt. Bei dieser Temperatur werden 125 ml Hexanlösung von 12,8 g n-Butyllithium zugetropft. Es entsteht unter exothermer Reaktion eine gelbe Lösung. Nach 15 Minuten Rühren werden 13,8 g Allendicarbonsäurediethylester in 100 ml THF bei -50°C zugetropft. Es entsteht eine rote Lösung, die bei -20°C 2 Stunden weitergerührt wird. Nach 2 Stunden wird das Reaktionsgemisch auf Zimmertemperatur erwärmt. Anschliessend werden zu dem Reaktionsgemisch 500 ml einer Puffer-Lösung (27 g $NH_4Cl$ gelöst in 100 ml konzentrierter Ammoniaklösung) zugegeben. Das Reaktionsgemisch wird 15 Minuten bei Zimmertemperatur gerührt, mit 500 ml gesättigter NaCl-Lösung verdünnt und mit 2 mal 400 ml Diethylether extrahiert. Die vereinten Extrakte werden mit $Na_2SO_4$ getrocknet und der Diethylether am Rotationsverdampfer abdestilliert. Der Destillationsrückstand (16,7 g) wird über 300 g $SiO_2$ chromatographiert (Laufmittel $CH_2Cl_2$, 0,3-0,5 bar Ueberdruck): Ausbeute 12,94 g.

Die Destillation des Produkts im Kugelrohrofen bei 120°C/1,3x$10^{-2}$ mbar liefert ein farbloses Oel.

b) 2-Butyl-3-ethoxymethylenyl-prop-1-en-1,3-dicarbonsäure-diethylester

11,34 g (0,06 mol) $TiCl_4$ in 15 ml $CCl_4$ werden unter Rühren bei 0°C zu 120 ml THF getropft. Zu dem entstandenen gelben Komplex werden nach 15 Minuten 4,44 g (0,06 mol) Ameisensäure-ethylester und anschliessend 3,63 g (0,015 mol) 2-Butyl-prop-1-en-1,3-dicarbonsäure-diethylester zugegeben. Nach 15 Minuten Rühren wird das Reaktionsgemisch bei 0°-5°C tropfenweise innerhalb 30 Minuten mit 12,12 g (0,12 mol) N-Methylmorpholin in 21 ml THF versetzt. Das Reaktionsgemisch wird 24 Stunden bei Zimmertemperatur gerührt und darauf in 150 ml $CH_2Cl_2$ und 100 ml gesättigte $KHCO_3$-Lösung eingetragen. Nach 30 Minuten Rühren wird das ausgeschiedene Titan-Salz abfiltriert. Aus dem Filtrat wird die $CH_2Cl_2$-Lösung vom Wasser getrennt. Die $CH_2Cl_2$-Phase wird mit Wasser gewaschen, mit $Na_2SO_4$ getrocknet, eingedampft und bei 100°C/1,3 mbar restliches N-Methylmorpholin entfernt. Der ölige Rückstand wird über 150 g $SiO_2$ (Laufmittel $CH_2Cl_2$, 0,3 bar Ueberdruck) filtriert. Das erhaltene rohe Produkt wird chromatographiert (Silikagel, Laufmittel: $CH_2Cl_2$). Man erhält 1,95 g (43,5 %) eines hellgelben Oels.

c) 4-Butyl-2H-2-oxy-pyran-5-carbonsäure-ethylester

1,4 g (0,0047 mol) 2-Butyl-3-ethoxy-methylenyl-prop-1-en-1,3-dicarbonsäure-diethylester und 14 ml Ameisensäure werden in einem auf 110°C vorerwärmten Bad 20 Minuten erhitzt. Die Ameisensäure wird dann im Wasserstrahlvakuum bei 50°C abdestilliert. Der Rückstand wird in 20 ml $CH_2Cl_2$ gelöst, die Lösung mit 10 ml gesättigter NaCl-Lösung gewaschen, mit $Na_2SO_4$ getrocknet und zur Trockene eingeengt. Ausbeute: 0,95 g (90,5 %) öliges Produkt.

Beispiel 2: 4-Benzylthio-2H-2-oxo-pyran-5-carbonsäureethylester

a) 2-Benzylmercapto-prop-1-en-1,3-dicarbonsäure-diethylester

1,84 g Na werden in 200 ml Methanol gelöst. Dazu werden 10,91 g Benzylmerkaptan zugefügt und das Methanol im Vakuum abdestilliert. Der Destillationsrückstand wird zweimal mit 100 ml Toluol eingedampft. Der Rückstand wird in 120 ml Dimethylsulfoxid (DMSO) gelöst und die Lösung mit 17,64 g β-Chlorglutaconsäure-diethylester versetzt. Das Reaktionsgemisch erwärmt sich auf 53°C. Es wird noch über Nacht bei Zimmertemperatur gerührt. Das DMSO wird im Vakuum bei 80°C/1,3 mbar abdestilliert. Der Rückstand wird in $CH_2Cl_2$ aufgenommen und die Lösung mit angesäuertem (HCl) Wasser gewaschen. Nach dem Trocknen mit $NaSO_4$ und Abdestillieren des $CH_2Cl_2$ erhält man 24,15 g Produkt (97.9 %) als goldgelbes Oel.

b) 2-Benzylmerkapto-3-ethoxymethyleno-prop-1-en-1,3-dicarbonsäure-diethylester

105,2 g (0,557 mol) $TiCl_4$ in 140 ml $CCl_4$ werden unter Rühren bei 0°C zu 1115 ml THF getropft. Anschliessend werden bei gleicher Temperatur zuerst 41,2 g (0,557 mol) Ameisensäure-ethylester und dann 43 g (0,139 mol) 2-Benzylmerkapto-prop-1-en-1,3-dicarbonsäure-diethylester zugefügt. Nach 15 Minuten wird bei gleicher Temperatur eine Lösung von 112,9 g (1,115 mol) N-Methylmorpholin in 195 ml THF während 30 Minuten zugetropft. Das Reaktionsgemisch wird dann bei Zimmertemperatur über Nacht gerührt und dann in ein Gemisch von 1000 ml $CH_2Cl$ und 2000 ml gesättigter $NaHCO_3$-Lösung eingetragen. Nach 30 Minuten Rühren wird das Gemisch filtriert. Die $CH_2Cl_2$-Phase des Filtrates wird von der wässrigen Phase getrennt, noch 2 mal mit 300 ml Wasser gewaschen, mit $Na_2SO_4$ getrocknet und im Vakuum eingeengt. Man erhält 47,66 g (94 %) eines orangen Oels.

c) 4-Benzylthio-2H-2-oxo-pyran-5-carbonsäure-ethylester

47,66 g (0,130 mol) 2-Benzylmerkapto-3-ethoxymethylenyl-prop-1-en-1,3-dicarbonsäure-ethylester wird in 481 ml Ameisensäure gelöst und in einem auf 110°C erwärmten Bad 20 Minuten erhitzt. Die Ameisensäure wird anschliessend im Vakuum abdestilliert. Der Rückstand wird in 500 ml $CH_2Cl_2$ aufgenommen, mit

wässriger NaCl-Lösung und anschliessend mit Wasser gewaschen. Nach dem Trocknen der Lösung mit $Na_2SO_4$ wird im Vakuum das Lösungsmittel abdestilliert. Der Destillationsrest kristallisiert aus [25,9 g (68,7 %)]. Der Schmelzpunkt beträgt (aus Ethanol umkristallisiert) 99-101°C.

Beispiel 3: 4-Chlor-2H-2-oxo-pyran-5-carbonsäure-ethylester

a) 1-Ethoxy-3-chlor-butadien-2,4-dicarbonsäure-diethylester

Zu 80 ml THF werden 7,56 g (0,04 mol) $TiCl_4$, gelöst in 10 ml $CCl_4$, bei 0°C getropft. Zum entstandenen gelben Komplex werden dann zuerst 2,96 g (0,04 mol) Ameisensäure-ethylester und darauf 2,2 g (0,01 mol) β-Chlorglutaconsäure zugefügt. Nach 15 Minuten wird bei 0°C eine Lösung von 8.08 g (0,08 mol) N-Methylmorpholin in 14 ml THF während 30 Minuten zugetropft. Das Reaktionsgemisch wird noch bei Zimmertemperatur über Nacht gerührt. Dann wird vom Reaktionsgemisch das Lösungsmittel bei 35°C im Vakuum abdestilliert. Der Rückstand wird in 200 ml $CH_2Cl_2$ aufgeschlämmt, 200 ml Wasser, das 11 g $NaHCO_3$ enthält, zugefügt und nach 1 Stunde Rühren filtriert. Die zwei Phasen des Filtrats werden getrennt und die organische Lösung im Vakuum einer Wasserstrahlpumpe eingeengt. Der Rückstand wird über eine Kolonne aus 35 g Silicagel (Laufmittel: $CH_2Cl_2$) filtriert. Nach dem Abdestillieren des Laufmittels bleiben 1,92 g (69,6 %) öliges Produkt.

b) 4-Chlor-2H-2-oxo-pyran-5-carbonsäure-ethylester

74,12 g (0,268 mol) 1-Ethoxy-3-chlor-butadien-2,4-dicarbonsäure-diethylester, gelöst in 700 ml Ameisensäure, werden in einem auf 110°C vorerwärmten Bad 30 Minuten erhitzt, abgekühlt und im Vakuum bei 50°C zur Trockene eingedampft. Der Rückstand wird in 250 ml $CH_2Cl_2$ aufgenommen, mit Wasser neutral gewaschen und nach dem Trocknen der Lösung mit $Na_2SO_4$ eingeengt. Der Destillationsrückstand wird über 1300 g Silikagel (Laufmittel: $CH_2Cl_2$-Aceton 99:1, 0,3 bar Ueberdruck) chromatographiert. Nach dem Einengen wird der Destillationsrückstand (29 g) aus einem Gemisch von 12 ml Diethylether und 10 ml Hexan bei -20°C kristallisiert. Ausbeute 22,79 g (42 %), Schmp.: 38°-39°C.

Beispiel 4: 4-Ethoxy-2-oxo-2H-pyran-5-carbonsäureethylester

a) 1,3-Ethoxy-butadien-2,4-dicarbonsäure-diethylester

Eine Lösung von 130,41 g (0,69 mol) $TiCl_4$ in 170 ml $CCl_4$ wird unter Rühren zu 1380 ml THF getropft. Die Temperatur wird dabei durch Kühlung auf 0°C gehalten. Danach werden zuerst 36,72 g (0,17 mol) 3-Ethoxy-glutaconsäure-ethylester, dann 51,06 g (0,69 mol) Ameisensäure-ethylester eingetragen und schliesslich tropfenweise innerhalb 0,5 Stunden bei 0°C 139,38 g (1,38 mol) N-Methylmorpholin in 241 ml THF zugegeben. Das Reaktionsgemisch wird über Nacht bei Zimmertemperatur gerührt, nachher in eine Lösung von 150 g $NaHCO_3$ in 1200 ml $H_2O$ und 1200 ml $CH_2Cl_2$ eingetragen und 2 Stunden gerührt. Das ausgefallene Titanoxid wird abgenutscht. Das Filtrat wird dann mit 3 x 300 ml Methylenchlorid extrahiert. Die vereinigten Methylenchlorid-Extrakte werden mit $Na_2SO_4$ getrocknet und eingeengt. Das zurückgebliebene Rohprodukt wird über eine Kolonne, gefüllt mit 1300 g Kieselgel 60 (Laufmittel $CH_2Cl_2$-Aceton 19:1, 0,3 bar Ueberdruck) chromatographiert. Nach Entfernen des Laufmittels erhält man 31,9 g (67,6 %) öliges Produkt.

b) 4-Ethoxy-2-oxo-2H-pyran-5-carbonsäure-ethylester

26 g (0,0908 mol) 1,3-Ethoxy-butadien-2,4-dicarbonsäure-diethylester und 156 g Polyphosphorsäure werden 30 Minuten auf 140°C erhitzt, mit 1000 ml Wasser verdünnt und mit zuerst 1000 ml und dann noch 3 mal 200 ml $CH_2Cl_2$ extrahiert. Die Extrakte werden mit gesättigter NaCl-Lösung bis zum pH 5 gewaschen. Die Lösung wird dann mit $Na_2SO_4$ getrocknet und eingedampft. Der Destillationsrückstand wird über 160 g Kieselgel 60 chromatographiert ($CH_2Cl_2$:Aceton 19:1, 0,3 bar Ueberdruck). Nach Entfernen des Laufmittels erhält man 11,3 g (58,7 %) Kristalle vom Schmelzpunkt 86-89°C.

Beispiel 5: 4,5-bis-Trifluormethyl-2-oxo-2H-pyran

50 g (0,208 mol) 2-Oxo-2H-pyran-4,5-dicarbonsäure-diethylester, 170 g (1,57 mol) $SF_4$ und 300 g (15 mol) HF werden 10 Stunden bei 150°C im Autoklaven erhitzt. Das Reaktionsgemisch wird mit 500 ml $CH_2Cl_2$ verdünnt und in 1500 ml Wasser gegossen. Der pH-Wert wird unter Rühren durch allmähliche Zugabe von $NaHCO_3$ auf 7,5 eingestellt. Die organische Phase wird abgetrennt, über $Na_2SO_4$ getrocknet, abfiltriert und das Lösungsmittel im Vakuum bei 50°C abdestilliert. Der Destillationsrückstand wird fünfmal mit 100 ml n-Pentan extrahiert und die Pentanlösung eingeengt. Nach Destillation bei 68-70°C/13 mbar werden 18,62 g (38,5 %) 4,5-bis-Trifluormethyl-2-oxo-2H-pyran erhalten.

Beispiel 6: 4-Fluor-2H-2-oxo-pyron-5-carbonsäureethylester

a) 3-Fluor-pent-2(E)-en-dicarbonsäurediethylester

Eine Mischung aus Tetrabutylammonium-dihydrogentrifluorid (hergestellt aus 30 mmol Tetrabutylammoniumfluorid, KF und HF), 20 ml 1,2-Dichlorethan und 15 mmol (2,76 g) Allendicarbonsäurediethylester wird 1 h unter Rückfluss erhitzt. Danach wird mit Methylenchlorid verdünnt und langsam mit Eiswasser, Natriumbicarbonat und Ammoniumchloridlösung gewaschen, mit $Na_2SO_4$ getrocknet und eingedampft. Das Rohprodukt

wird mit 500 ml Hexan/Essigester (4:1) über 50 g Kieselgel flash-filtriert und am Hochvakuum destilliert. Man erhält 1,79 g (59 % d. Th.) des gewünschten Produkts; Siedepunkt: 50-55° C/0,06 mbar.

b) 1-Ethoxy-3-fluor-butadien-2,4-dicarbonsäure-diethylester

Ein Gemisch von 3,78 g (0,02 mol) $TiCl_4$ in 5 ml $CCl_4$ wird in 40 ml Tetrahydrofuran (THF) unter Rühren bei 0° C zugetropft. Nachher werden noch 1,02 g (0,005 mol) 3-Fluor-pent-2(E)-en-dicarbonsäure-diethylester und 1,48 g (0,02 mol) Ameisensäureethylester zugetropft. Nach 15 Minuten wird bei 0-5° C noch eine Lösung von 4,04 g (0,04 mol) N-Methylmorpholin in 7 ml THF zugetropft. Das Reaktionsgemisch wird 17 h bei Raumtemperatur gerührt und dann in ein Gemisch von 50 ml gesättigter $KHCO_3$-Lösung und 100ml $CH_2Cl_2$ eingerührt. Das ausgefallene Titansalz wird abgesaugt und die organische Phase von der wässrigen Phase abgetrennt. Die wässrige Phase wird noch einmal mit 100 ml $CH_2Cl_2$ extrahiert und die vereinten organischen Phasen mit $Na_2SO_4$ getrocknet und eingeengt. Der Destillationsrückstand wird bei 85° C/1,3 mbar von überschüssigem N-Methylmorpholin befreit und der Rest über 150 g $SiO_2$ (Laufmittel $CH_2Cl_2$-Aceton 19:1) chromatographiert. Man erhält 1 g (77 % d. Th.) des Produktes (oranges Oel).

c) 4-Fluor-2H-2-oxo-pyran-5-carbonsäure-ethylester

1 g (0,0038 mol) 1-Ethoxy-3-fluor-butadien-2,4-dicarbonsäure-diethylester wird mit 6 g PPA (Polyphosphorsäure) versetzt und 30 Minuten bei 80° C gerührt, mit 20 ml Wasser verdünnt und 3 mal mit 20 ml $CH_2Cl$ extrahiert. Die $CH_2Cl_2$-Phase wird mit $H_2O$ gewaschen, getrocknet ($Na_2SO_4$) und eingeengt. Der Destillationsrückstand wird über Silicagel chromatographiert (Laufmittel $CH_2Cl_2$-Aceton 99:1). Nach dem Eindampfen des Lösungsmittels bleibt als Rückstand 0,2 g (28 % d. Th.) des reinen Produktes (oranges Oel).

Beispiel 7: 4-Methylsulfonyl-2H-2-oxo-pyron-5-carbonsäureethylester

a) 3-Methylsulfonyl-pent-2-endicarbonsäurediethylester

Eine Mischung von 0,44 g (0,002 mol) 3-Chlor-pent-2-en-dicarbonsäure-diethylester und 0,3 g (0,003 mol) Natriumsulfinat in 1,5 Dimethylformamid (DMF) wird bei 100° C 1 Stunde gerührt und darauf DMF im Vakuum bei 110° C/1,3 mbar abdestilliert. Der Rückstand wird mit 0,5 ml Wasser vermischt. Der in Wasser unlösliche Anteil kristallisiert aus und wird abgesaugt und mit Wasser nachgewaschen. Das rohe kristalline Material wird über 12 g Silikagel chromatographiert (Laufmittel $CH_2Cl_2$/Aceton 99:1). Man erhält 0,43 g (81,44 % d. Th.) des Produktes; Schmelzpunkt: 54-57° C.

b) 1-Ethoxy-3-methylsulfonyl-butadien-2,4-dicarbonsäure-diethylester

In 48 ml THF wird eine Lösung von 4,53 g (0,024 mol) $TiCl_4$ in 6 ml $CCl_4$ unter Kühlung mit Eis (0-5° C) zugetropft. Bei der gleichen Temperatur wird dann unter Rühren zuerst 1,77 g (0,024 mol) Ameisensäureester und dann 1,58 g (0,006 mol) 3-Methylsulfonyl-pent-2-endicarbonsäurediethylester zugetropft. Nach 30 Minuten wird während 1 Stunde bei 0-5° C noch 4,86 g (0,048 mol) N-Methylmorpholin in 8,5 ml THF zugefügt. Das Reaktionsgemisch wird dann 20 Stunden bei 25° C gerührt, mit 100 ml $CH_2Cl_2$ verdünnt und in 30 ml gesättigte $KHCO_3$-Lösung gegossen. Das Titansalz wird abgenutscht und aus dem Filtrat die organische Phase getrennt. Die wässrige Phase wird noch mit 50 ml $CH_2Cl_2$ extrahiert. Die vereinten $CH_2Cl_2$-Extrakte werden mit gesättigter NaCl-Lösung gewaschen, mit $Na_2SO_4$ getrocknet und eingeengt. Der Destillationsrückstand wird chromatographiert (200 g Silikagel, Laufmittel $CH_2Cl_2$-Aceton 19:1). Man erhält 1,17 g (60,94 %) des Produktes.

c) 4-Methylsulfonyl-2-oxo-2H-pyran-5-carbonsäure-ethylester

In eine DMF-Lösung von 2,02 g 4-Chlor-2-oxo-2H-pyran-5-carbonsäure-ethylester wird 1,2 g Methylsulfinsäurenatrium eingetragen und 2 h bei Raumtemperatur gerührt. Das Reaktionsgemisch wird mit $CH_2Cl_2$ verdünnt und mit gesättigter wässriger NaCl-Lösung extrahiert. Die organische Phase wird mit $Na_2SO_4$ getrocknet und eingedampft. Der auskristallisierte Destillationsrückstand wird mit Ether vermischt, abgesaugt und mit Ether nachgewaschen. Ausbeute 1,4 g (56,9 % d. Th.); Schmelzpunkt: 95-97° C.

Beispiel 8: 6,7-bis-Trifluormethyl-1,4-naphthochinon

23,47 g (0,1011 mol) 4,5-bis-Trifluormethyl-2-oxo-2H-pyran, 54,62 g (0,5053 mol) 1,4-Naphthochinon und 26,30 g (0,3026 mol) $MnO_2$ werden in 225 ml 1,2-Dichlorbenzol 12 Stunden auf 180°C erhitzt. Das Reaktionsgemisch wird anschliessend mit 200 ml $CHCl_3$ verdünnt und über Hyflo® filtriert. Nach dem Abdestillieren von Chloroform wird der Destillationsrückstand über eine mit 1300 g $SiO_2$ gefüllte Kolonne chromatographiert (Laufmittel: Zuerst Cyclohexan, dann $CHCl_3$; 0,3 bar Ueberdruck). Ausbeute 17,94 g (60,3 %), Schmelzpunkt: 91-94° C.

Beispiel 9: 6-Ethoxy-1,4-naphthochinon-7-carbonsäure-ethylester

0,42 g (0,002 mol) 4-Ethoxy-2-oxo-2H-pyran-5-carbonsäure-ethylester, 1,08 g (0,01 mol) 1,4-Benzochinon und 0,54 g (0,0062 mol) $MnO_2$ werden in 4,8 ml Dichlorbenzol 12 Stunden auf 180° C erhitzt. Das erhaltene Reaktionsgemisch wird über eine Kolonne von 30 g Kieselgel 60 (Laufmittel $CH_2Cl_2$:Aceton 99:1, 0,3 bar Ueberdruck) chromatographiert. Man erhält 0,41 g (75,9 %) Kristalle vom Schmelzpunkt 60-62° C.

Anwendungsbeispiel

a) Herstellung von 2,3-Di(trifluormethyl)-5,6,11,12-tetrathiotetracen

25 mMol 6,7-Bis-trifluormethyl-1,4-naphthochinon, ca. 37 mMol 1,2-Dibrombenzocyclobuten [siehe J. Am. Chem. Soc., 79, S. 1701 ff (1957)], das etwas 1-Brom-2-iod-benzocyclobuten enthält, und 100 ml Xylol werden während 16 Stunden mit einem Wasserabscheider am Rückfluss gehalten. Das Reaktionsgemisch wird abgekühlt, der Niederschlag abfiltriert und mit Xylol gewaschen. Man erhält das kristalline 2,3-Di(trifluormethyl)naphthacen-5,12-dion in einer Ausbeute von 5,82 g (71 %) mit einem Schmelzpunkt von > 280° C.

1,65 mMol 2,3-Di(trifluormethyl)naphthacen-5,12-dion, 5 ml Essigester, 4,96 mMol Kaliumacetat und 3 ml Acetanhydrid werden unter Zugabe von 0,1 g Pd/C (5 %) bei 20-25° C während 35 Minuten hydriert. Das Reaktionsgemisch wird filtriert und der Rückstand dreimal mit $CH_2Cl_2$ gewaschen. Die Filtrate werden eingedampft und der Rückstand aus $CH_2Cl_2$/Pentan umkristallisiert. Man erhält 2,3-Di(trifluormethyl)-5,12-diacetoxynaphthacen in einer Ausbeute von 0,45 g (70 %), Schmelzpunkt 149-153° C.

In einem 100 ml Kolben mit Rückflusskühler und Gaseinleitungsrohr werden unter leichtem Argonfluss 0,61 mMol des Diacetoxynaphthacens, 2,43 mVal $S_8$ und 0,01 mMol p-Toluolsulfonsäure in 35 ml 1,2,4-Trichlorbenzol 20 Stunden refluxiert. Nach dem Abkühlen wird am Hochvakuum das Lösungsmittel abgedampft, der Rückstand mit Hexan ausgekocht, das schwarze Pulver abfiltriert und am Hochvakuum bei 60° C getrocknet. Man erhält 79 % Rohrprodukt. Dieses wird bei 190° C (1,3x$10^{-4}$ mbar) sublimiert, wobei 75,6 mg (30 %) reines 2,3-Di(trifluormethyl)-5,6,11,12-tetrathiotetracen als schwarze Nädelchen erhalten werden. Massenspektrum: $M^+ = 488$; $\lambda_{max}$ (1,2,4-Trichlorbenzol): 755 nm.

b) Elektrochromie

In eine Elektrochromiezelle, bestehend aus einer Teflonmembran und in je 0,5 mm Abstand einer Anode und Kathode aus ITO-Glas, werden zur Anodenseite 1 mg 2,3-Di(trifluormethyl)-5,6,11,12-tetrathiotetracen und 100 mg $LiClO_4$, gelöst in 5 ml Aceton, und zur Kathodenseite eine Lösung von 1 mg 2,3-Di(trifluormethyl)-5,6,11,12-tetrathiotetracen-perchlorat (CT-Komplex, Herstellung siehe US-PS 3,634,336) und 100 mg $LiClO_4$ in 5 ml Aceton eingefüllt. Nach Anlegen einer Spannung von 2 Volt wechseln innerhalb weniger Sekunden die Farben von grün nach rotviolett auf der Anodenseite und von rotviolett nach grün auf der Kathodenseite. Durch Umpolung der Spannung werden in beiden Zellenhälften die ursprünglichen Farben erhalten. Der gleiche Effekt wird beobachtet, wenn man Nitrobenzol oder Dimethylformamid als Lösungsmittel verwendet.

**Patentansprüche**

1. Verbindungen der Formel I

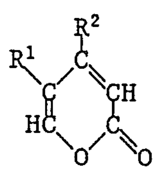

(I),

worin

$R^1$ für $-CF_3$ oder $-COOR^3$ steht und $R^3$ den um eine Hydroxylgruppe verminderten Rest eines $C_1$-$C_{18}$-Alkohols bedeutet, und $R^2$ -F, -Br, -Cl, -CN, $-CF_3$, $C_1$-$C_{18}$-Alkyl, $C_2$-$C_{18}$-Alkenyl oder -Alkinyl, $C_1$-$C_{18}$-Alkoxy, $C_1$-$C_{18}$-Alkylthio, $C_1$-$C_{18}$-Alkylsulfonyl, $C_6$-$C_{16}$-Aryl, $C_7$-$C_{24}$-Alkaryl, $C_7$-$C_{12}$-Aralkyl, $C_8$-$C_{24}$-Alkaralkyl, $C_6$-$C_{16}$-Aryloxy oder -thio oder -sulfonyl, $C_7$-$C_{24}$-Alkaryloxy oder -thio oder -sulfonyl, $C_7$-$C_{12}$-Aralkyloxy oder -thio oder -sulfonyl oder $C_8$-$C_{24}$-Alkaralkyloxy oder -thio oder -sulfonyl, Sekundäramino mit 2 bis 24 C-Atomen, oder Trialkyl- oder Trialkoxysilyl mit 3 bis 18 C-Atomen darstellt.

2. Verbindungen gemäss Anspruch 1, worin $R^3$ $C_1$-$C_{18}$-Alkyl bedeutet.

3. Verbindungen gemäss Anspruch 2, worin $R^3$ für Methyl oder Ethyl steht.

4. Verbindungen gemäss Anspruch 1, worin $R^2$ für -F, -CN, $-CF_3$, -Br, -Cl, $C_1$-$C_{18}$-Alkyl oder $C_2$-$C_{12}$-Alkenyl, $C_1$-$C_{18}$-Alkoxy, $C_1$-$C_{18}$-Alkylthio, $C_1$-$C_{18}$-Alkylsulfonyl, Phenyl, $C_1$-$C_{12}$-Alkylphenyl, Phenyl-$C_nH_{2n}$-, $C_1$-$C_{12}$-Alkylphenyl-$C_nH_{2n}$-, Phenyloxy, Phenylthio oder Phenylsulfonyl, $C_1$-$C_{12}$-Alkylphenoxy, -phenylthio oder -phenylsulfonyl, Phenyl-$C_nH_{2n}$-O-, Phenyl-$C_nH_{2n}$-S- oder Phenyl-$C_nH_{2n}$-$SO_2$-, $C_1$-$C_{12}$-Alkylphenyl-$C_nH_{2n}$-O-, $C_1$-$C_{12}$-Alkylphenyl-$C_nH_{2n}$-S- oder $C_1$-$C_{12}$-Alkylphenyl-$C_nH_{2n}$-$SO_2$-, wobei n eine Zahl von 1 bis 4 ist, Sekundäramino mit 2 bis 18 C-Atomen, oder Trialkyl- oder Trialkoxysilyl mit 3 bis 12 C-Atomen steht.

5. Verbindungen gemäss Anspruch 1, worin $R^2$ für -F, -Cl, -Br, $-CF_3$, $C_1$-$C_{12}$-Alkyl, $C_1$-$C_{12}$-Alkoxy, $C_1$-$C_{12}$-Alkylthio, Benzylthio oder $C_1$-$C_{12}$-Alkylbenzylthio, oder Sekundäramino mit 2 bis 12 C-Atomen steht.

6. Verfahren zur Herstellung von Verbindungen der Formel I gemäss Anspruch 1, dadurch gekennzeichnet, dass man eine Verbindung der Formel II

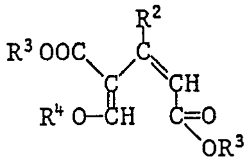

(II),

worin $R^2$ und $R^3$ die in Anspruch 1 angegebenen Bedeutungen haben, und $R^4$ für $C_1$-$C_4$-Alkyl steht, in Gegenwart einer starken wasserfreien Säure zu einer Verbindung der Formel I cyclisiert.

7. Naphthochinone der Formel VI

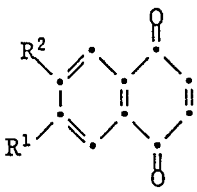

(VI),

worin $R^1$ und $R^2$ die in Anspruch 1 angegebenen Bedeutungen haben.

8. Naphthochinone gemäss Anspruch 7, worin $R^2$ -F, $-CF_3$, -Cl, -Br, $C_1$-$C_{18}$-Alkyl, -Alkoxy, -Alkylthio oder-Alkylsulfonyl, Phenyloxy oder -thio oder -sulfonyl, $C_1$-$C_{12}$-Alkylphenyloxy oder -thio oder -sulfonyl, Benzyloxy oder -thio oder -sulfonyl, oder $C_1$-$C_{12}$-Alkylbenzyloxy oder -thio oder -sulfonyl ist.

9. Naphthochinone gemäss Anspruch 7, worin $R^1$ für $-CF_3$, oder $-COOR^3$ und $R^2$ für $-CF_3$, -F, -Cl, $C_1$-$C_{12}$-Alkyl, -Alkoxy, oder -Alkylthio oder Alkylsulfonyl oder Benzylthio und $R^3$ für $C_1$-$C_4$-Alkyl stehen.

10. Verbindungen der Formel II

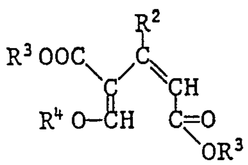

(II),

worin $R^2$ und $R^3$ die in Anspruch 1 angegebenen Bedeutungen haben, und $R^4$ für $C_1$-$C_4$-Alkyl steht.

11. Verbindungen der Formel II gemäss Anspruch 10, worin $R^2$ -F, $-CF_3$, -Cl, -Br, $C_1$-$C_{18}$-Alkyl, -Alkoxy, -Alkylthio oder-Alkylsulfonyl, Phenyloxy oder -thio oder -sulfonyl, $C_1$-$C_{12}$-Alkylphenyloxy oder -thio oder -sulfonyl, Benzyloxy oder -thio oder -sulfonyl, oder $C_1$-$C_{12}$-Alkylbenzyloxy oder -thio oder -sulfonyl ist.

12. Verbindungen der Formel II gemäss Anspruch 10, worin $R^1$ für $-CF_3$ oder $-COOR^3$ und $R^2$ für $-CF_3$, -F, -Cl, $C_1$-$C_{12}$-Alkyl, -Alkoxy oder -Alkylthio oder -Alkylsulfonyl oder Benzylthio und $R^3$ für $C_1$-$C_4$-Alkyl stehen.

13. Verbindungen der Formel II gemäss Anspruch 10, worin $R^4$ für Methyl oder Ethyl steht.